Europäisches Patentamt

(19) European Patent Office

Office européen des brevets

(11) Publication number: **0 454 338 A1**

(12) **EUROPEAN PATENT APPLICATION**

(21) Application number: 91303348.6

(22) Date of filing: 16.04.91

(51) Int. Cl.⁵: **C12P 21/08**, C12N 5/12, G01N 33/577

(30) Priority: 16.04.90 US 509890

(43) Date of publication of application:
30.10.91 Bulletin 91/44

(84) Designated Contracting States:
AT BE CH DE ES FR GB IT LI NL

(71) Applicant: DUKE UNIVERSITY
Erwin Road
Durham North Carolina 27706 (US)

(72) Inventor: Bast, Robert C., Jr.
6108 Turkey Farm Road
Chapel Hill, North Carolina 27514 (US)
Inventor: Iglehart, J. Dirk
703 Watts Street
Durham, North Carolina 27701 (US)
Inventor: Deutsch, Margaret A.
605 Jones Ferry Road, Apartmetn DD-9
Carrboro, North Carolina 27510 (US)
Inventor: Pence, Jeffrey C.
7519 Citadel Drive
College Park, Maryland 20740 (US)

(74) Representative: Horner, Martin Grenville et al
Cruikshank & Fairweather 19 Royal Exchange
Square
Glasgow G1 3AE Scotland (GB)

(54) **Method of diagnosing cancer.**

(57) A method of diagnosing cancer with antibodies directed to non-cellular components in non-vascular, cellular secretions is disclosed. One such antibody is the monoclonal antibody Lu4B5, which was produced by immunizing mice with a purified, mucin containing fraction prepared from pooled bronchial washings from adenocarcinoma and large cell lung cancer patients. Lu4B5 monoclonal antibody reacts with the bronchial secretions of patients with primary bronchogenic carcinoma significantly more frequently than with secretions from patients without primary lung cancer.

EP 0 454 338 A1

## Field of the Invention

The present invention concerns the use of soluble antigens contained in nonvascular secretions for detecting cancer in a human or animal subject.

## Background of the Invention

Bronchogenic carcinoma of the lung continues to be the leading cause of death resulting from cancer in the United States (Cancer Facts and Figures 1980, New York, 1979, American Cancer Society). The most effective treatment for lung cancer appears to be surgical removal of the tumor. Unfortunately, less than half of all lung cancer patients have tumors which can be surgically removed at the time of diagnosis. Accordingly, there is a need for more rapid and accurate means for diagnosing lung cancer.

P. Linsley et al., EPO Pat. Appln. No. 268279, published 25 May 1988, concerns hybridomas which produce monoclonal antibodies to mucin epitopes. The mucins are obtained from purified milk or from pleural effusions from cancer patients. Milk was obtained from normal, cancer-free donors (page 5 lines 26-27). It is not suggested that secretions be collected from a cancer patient. Use of the mucin monoclonal antibodies to bind bronchial brushings obtained during bronchoscopic examination is suggested (See Example IX at page 26).

J. Mattes et al., U.S. Patent No. 4,863,854, issued 5 September 1989, concerns monoclonal antibodies to mucin-like antigens, which antigens are suggested to be useful for diagnosing various cancers. The antibodies were produced by immunizing mice with carcinoma cell lines (col. 2 lines 67-68). Antibody MT334 reportedly bound to an antigen in the cytoplasm of carcinoma cell lines from ovary, colon, lung, pancreas, prostate, bladder, breast, liver and bile duct (col. 6 line 51 - col. 7 line 4). The antigen is suggested to be a mucin-like molecule which is secreted by cells. In frozen tissue sections, Ab MT334 reportedly bound to an antigen concentrated on the luminal side of mucus-producing goblet cells in the base of the glands of the colon (col. 7, lines 5-10). Antibody MQ49 binds to an antigen suggested to be a sulfated mucin-like molecule. On cell lines, MQ49 was detected on the surface of ovarian, renal colon, breast, lung, uterine and pancreatic carcinomas and a teratocarcinoma (Col. 7, lines 39-43). In frozen tissue sections, MQ49 was detected in secretory epithelial cells, including sweat glands of the skin, mucous glands of the stomach and epithelial cells of the colon, pancreas, esophagus, ureter and breast (Col. 7, lines 48-53).

J. Taylor-Papadimitriou et al., PCT Pat. Appln. No. WO 88/05054, published 14 July 1988, concerns an antibody against a human polymorphic epithelial mucin (HPEM) core protein, which antibody or fragment has reduced or substantially no reaction with fully processed HPEM. The mucin core protein was isolated from human milk (see pages 21-27). Antibodies to the core protein are stated to react with HPEM core protein, especially as expressed by colon, lung, ovary and breast carcinomas (see page 4).

R. Ceriani and J. Peterson, PCT Pat. Appln. No. WO 89/07268, published 10 August 1989, concerns a monoclonal antibody which binds to an antigen determinant on the surface of human breast carcinoma cells but which does not bind to normal human breast tissue. The antigen is characterized as a high molecular weight mucin-like glycoprotein complex having a molecular weight exceeding 400 Kd. The antibody is stated to recognize adenocarcinoma cells of the breast, ovary, endometrium, lung and pancreas (See page 3, lines 2-4).

A. Linnane, U.S. Patent No. 4,818,682, dated 4 April 1989, discloses an in vitro diagnostic method for detecting the presence of cells producing an intestinal mucin antigen in cancer patients. The method comprises testing a sample of blood, blood serum or blood plasma taken from the patient to detect the presence of small intestine mucin antigen (SIMA) and/or large intestine mucin antigen (LIMA) in the sample.

I. Hellstrom et al., U.S. Patent No. 4,906,562, dated 6 March 1990, concerns monoclonal antibodies which bind to a glycolipid antigen associated with human non-small cell lung carcinomas.

While monoclonal antibodies to some cancer antigens are known, it is also known that there are a variety of different types of tumors, and that the effective diagnosis of cancer remains a problem. The present invention is based on our ongoing research in this area.

## Summary of the Invention

This invention may be viewed as a unique approach to screening for cancer, the diagnosis of cancer, or the localization of cancer (particularly lung cancer). The invention concerns the use of the non-cellular, chemical components of secretions which can be analyzed immunologically with antibody as a marker for populations at risk to develop cancer or to diagnose cancer in patients suspected of harboring a malignancy. Furthermore, the invention concerns the use of antibodies to non-cellular, chemical components of secretions, particularly lung secretions, as a marker for patients at risk to develop lung cancer.

A first aspect of the present invention is a monoclonal antibody selected from the group consisting of monoclonal antibody Lu4B5, monoclonal antibodies which bind to the antigen (or more preferably the epitope) bound by monoclonal antibody Lu4B5, and fragments thereof which bind to the antigen (or more preferably the epitope) bound by monoclonal antibody Lu4B5.

A second aspect of the present invention is a cell line capable of producing a monoclonal antibody as described above.

A third aspect of the present invention is a method for detecting the presence of cancer. The method comprises contacting a sample from a subject with a monoclonal antibody as described above under conditions permitting the monoclonal antibody to form a reaction product, and then detecting the presence or absence of the reaction product. The method may be conducted with any suitable assay, including both homogeneous and heterogeneous (e.g., sandwich) assays.

Antibodies like those of the present invention may be made by collecting mucus secretions from a subject; then providing a non-cellular phase produced from the collected mucus secretions; then generating a plurality of antibodies against antigens contained in the non-cellular phase; and then selecting antibodies from said plurality of antibodies, which selected antibodies are capable of selectively detecting cancer or a risk of cancer in a subject. The mucus secretions are preferably aero-digestive tract mucus secretions, and are preferably collected from cancerous tissue. Most preferably the mucus secretions are respiratory tract secretions from a patient afflicted with lung cancer. The non-cellular phase prepared from the collected secretions is preferably mucin-containing.

## Detailed Description of the Invention

This invention, in one respect, provides a monoclonal antibody (Lu4B5) which detects chemical non-cellular components in the fluid phase of pulmonary secretions and is capable of distinguishing patients with primary bronchogenic carcinoma from those individuals who do not have primary lung cancer. This antibody was raised against non-cellular chemical components of mucous secretions.

The use of mucous secretions to develop immunological reagents to diagnose epithelial cancers is unique. Mucous non-vascular secretions are distinguished from other body fluids which have been used to develop immunological reagents by several specific and widely accepted definitions: (1) mucous secretions are produced by endodermal (glandular) cells, the mucous membranes, which line body cavities and which are in communication with the external world; (2) the production of mucous secretions is a function of mucous membranes and requires metabolic energy, in contrast to filtrates (central nervous system fluid) and exudates (serous effusions of internal body spaces, e.g., inflammatory or malignant effusions); (3) mucous secretions are considered external fluids which bath the mucous membranes of body cavities which are in direct communication with the external world; (4) the mucous secretions contain primarily high molecular weight mucins, as well as proteins, which provide a vehicle to carry other products (cells, milk proteins, debris, feces, etc.) to the outside world; (5) The mucous secretions cling to mucous membranes and serve to protect these surfaces from toxic chemicals and microorganisms.

Specific examples of non-vascular, non-cellular mucous secretions are the secretions of the aero-digestive tract (including the oro-pharynx, the throat, the respiratory tree, the esophagus, the stomach and intestines), the breast (secretions within breast ducts and acini), and the genito-urinary tract (secretions of the bladder, ureters, kidneys, prostate, vagina, cervix, and uterus) of humans. Aero-digestive tract secretions are preferred for carrying out the present invention, and respiratory tract secretions (e.g., secretions of the respiratory tree) are particularly preferred.

The Lu4B5 antibody, affixed to a solid supporting matrix, has discovered a large complex glycoprotein (mucin) within the nonvascular and non-cellular secretions of the lung and produced by certain cell lines. This glycoprotein, defined by its possession of epitopes which bind with Lu4B5, is produced by normal, non-cancerous, epithelial cells which line parts of the aero-digestive tract of humans (normal colon epithelial cells and epithelial cells in the respiratory tract of certain people who have primary bronchogenic carcinoma). The mucin or glycoprotein molecule which has been discovered by its binding to Lu4B5 was contained in the flow-through fraction after separation of the chemical components of pooled and lyophilized and resuspended (non-cellular) mucous secretions of the lung applied to a molecular size exclusion column (Sephacryl S400, Pharmacia, Inc.). In principle, chemical separation of non-vascular and non-cellular mucous secretions by size exclusion chromatography combined with production of antibodies, either polyclonal, monoclonal, or recombinant antibodies, can be used to identify cancer associated, novel epitopes or glycoproteins which will distinguish persons bearing primary malignancies of the normal cells which produce these mucins or glycoproteins. Such immunological reagents can then be used in standard immunological assay formats (radioimmunoassay, immunoenzyme linked assays) to identify patients with primary epithelial malignancy of the cells which produce these mucins or glycoproteins or patients who are at increased risk to develop malignancies of the epithelial cells which produce these mucins or glycoproteins. As such, this invention concerns the isolation of molecules (proteins, glycoproteins, mucins) which are produced by epithelial cells and shed into the non-vascular and non-cellular mucous secretions within body cavities lined by these epithelial cells combined with the production of antibodies (polyclonal, monoclonal, or recombinant antibodies) to these molecules which will identify patients with malignancies of epithelial cells lining these body cavities or people who are at increased risk to develop malignancies of the epithelial cells which line body cavities and secrete these mucous fluids.

Another aspect of the present invention is the production of antibodies (polyclonal, monoclonal, or recombinant) which are made using the non-cellular components of mucous secretions (defined above) as the immunizing agent.

The hybridomas and monoclonal antibodies of the invention may be produced according to the technique of Kohler and Milstein, *Nature* **265**, 495-97 (1975). For example, non-vascular secretions may be injected into a mouse and, after a sufficient time, the mouse sacrificed and spleen cells obtained. The spleen cells are then immortalized by fusing them with myeloma cells or with lymphoma cells, typically in the presence of polyethylene glycol, to produce hybridoma cells. The hybridoma cells are then grown in a suitable media and the supernatant screened for monoclonal antibodies having the desired specificity.

Antibody fragments included within the scope of the present invention include, for example, Fab, F(ab')$_2$, and Fv fragments.

Techniques for generating monoclonal Fab fragments in *Escherichia coli* are described in W. Huse, *Generation of a Large Combinatorial Library of the Immunoglobulin Repertoire in Phage Lamda, 246 Science* 1275 (8 Dec. 1989).

Another aspect of the present invention is the use of antibody based tests applied to the non-vascular mucous secretions of patients suspected of having epithelial malignancies of the mucous membranes which secrete these fluids, regardless of the source of the antibodies. In addition, the antibodies discussed herein may be used to test the non-vascular mucous secretions of patients who have been found free of cancer to determine whether that patient is at risk of developing cancer.

Examples of tests which can be applied to non-vascular, non-cellular mucous secretions include immunological detection of proteins, glycoprotein, and mucins by radioimmunoassay or enzyme-linked antibody assays of non-vascular, non-cellular secretions bound to solid supports or molecules soluble within fluid extracts of these secretions. Because the molecules are produced by non-malignant, morphologically normal epithelial cells, it is possible to apply detection of epitopes on these molecules to identification of patients with epithelial malignancies or to identification of patients without pathologically defined carcinoma who may be at increased risk to develop malignancies of the epithelial cells which produce these non-vascular, non-cellular secretions.

Assays carried out in accordance with the present invention may be homogeneous or heterogeneous. In a homogeneous assay approach, the specimen may be a biological fluid such as a mucus sample, bronchial washings, serum, urine, and the like or the specimen may be tissue which is lysed and clarified to remove debris. The immunological reaction usually involves the specific antibody (e.g., Lu4B5), a labeled analyte, and the sample of interest. The signal arising from the label is modified, directly or indirectly, upon the binding of the antibody to the labeled analyte. Both the immunological reaction and detection of the extent thereof are carried out in a homogeneous solution. Immunochemical labels which may be employed include free radicals, radioisotopes, fluorescent dyes, enzymes, bacteriophages, coenzymes, and so forth. In a heterogeneous assay approach, the reagents are usually the specimen, the antibody of the invention (e.g., Lu4B5), and means for producing a detectable signal. The specimen is generally immobilized on a support, such as a plate or a slide, and contacted with the antibody in a liquid phase. The support is then separated from the liquid phase and either the support phase or the liquid phase is examined for a detectable signal employing means for producing such signal. The signal is related to the presence of the analyte in the specimen. Means for producing a detectable signal includes the use of radioactive labels, fluorescers, enzymes, and so forth. Examplary of heterogeneous immunoassays are the radioimmunoassay, immunofluorescence methods, enzyme-linked immunoassays, and the like. See generally "Enzyme-Immunoassay," by Edward T. Maggio, CRC Press, Inc., Boca Raton, Fl., 1980; see also U.S. Pat. Nos. 4,906,562, 4,863,854, and 4,818,682, the disclosures of which are incorporated herein by reference.

Monoclonal antibodies as described herein may conveniently be used in a "two-site" or "sandwich" assay, with a single cell line serving as a source for both the labeled monoclonal antibody and the bound monoclonal antibody. Such assays are described in U.S. Patent No. 4,376,110, the disclosure of which is incorporated herein by reference.

The invention also includes diagnostic kits for carrying out the methods disclosed above. In one embodiment, the diagnostic kit comprises (a) an antibody of the invention (e.g., Lu4B5) and (b) a conjugate of a specific binding partner for the above monoclonal antibody and a label capable of producing a detectable signal. The reagents may also include ancillary agents such as buffering agents and protein stabilizing agents, e.g., polysaccharides and the like. The diagnostic kit may further include, where necessary, other members of the signal-producing system of which system the label is a member, agents for reducing background interference in a test, control reagents, apparatus for conducting a test, and the like. In another embodiment, the diagnostic kit comprises a conjugate of an antibody of the invention (e.g., Lu4B5) and a label capable of producing a detectable signal. Ancillary agents as mentioned above may also be present.

The present invention is described in further detail in the following Examples. These Examples are illustrative, and are not to be taken as limiting of the invention.

## EXAMPLE 1

### Preparation of Fluid Phase of Bronchial Secretions

Bronchial washings were obtained from four groups of patients. The patient groups were as follows: (1) lung adenocarcinoma/large cell carcinoma patients; (2) lung squamous cell carcinoma patients; (3) non-cancer patients who were heavy smokers (> 20 pack years); and (4) non-smoking patients. Bronchial washings were obtained from patients by the instillation and aspiration of 20 milliliters of sterile saline according to known procedures. See J.D. Iglehart et al., 82 J. Thorac. Cardiovasc. Surg. 63 (1981). In some cases, in place of a bronchial washing, induced sputum was obtained from volunteer patients by causing them to breath warm humidified air as an irritant. The bronchial washings (or induced sputum) were pooled by group to provide four pools of washings. 10 milliliters from each pool, diluted in phosphate-buffered saline, was purified by column chromatography on a Sephacryl S400 column. The fraction close to the void volume was selected as the high molecular weight fraction and used for the production and selection of monoclonal antibodies.

## EXAMPLE 2

### Preparation of Lu4B5 Hybridoma Cell Line

The Lu 4B5 hybridoma cell line was developed from fusion of NS-1 myeloma cells with spleen cells of Balb/C mice immunized with the high molecular weight fraction of pooled sputum specimens from the adenocarcinoma/large cell lung cancer group prepared as described in Example 1 above. The resultant antibodies were screened for selective binding to the pooled lung cancer sputum specimen used to immunize the mice, and for lack of significant binding to the corresponding high molecular weight fraction of pooled sputum specimen from the non-smoking patient group described in Example 1 above. This screening was accomplished with an indirect immunofluorescence assay, utilizing the Pandex screen machine (Baxter, Chicago, Illinois). Reactivity to pooled high molecular weight fractions sputum specimens from the smoking patient group was also performed. The selected group of antibodies were isotyped utilizing an immunofluorescence assay developed for the Pandex screen machine.

The Lu 4B5 hybridoma cell was selected from 791 clones derived from four fusions of NS-1 myeloma cells with Balb/c mouse spleen cells. The cell line was selected based on its production of an $IgG_1$ antibody which reacted with the high molecular weigh fraction used to immunize the mouse but not to a high molecular weight fraction of pooled sputum from non-smoking individuals. Intermediate binding was noted to the high molecular weight fraction of pooled sputum from the heavy smoking group. Hybridoma Lu 4B5 was deposited at the American Type Culture Collection, Rockville, Md. 20852 USA in accordance with the provisions of the Budapest Treaty on April 17, 1990, and has been assigned ATCC Number HB 10425.

## EXAMPLE 3

### Production of Lu4B5 Antibody from Lu4B5 Cell Line

Milligram amounts of the selected antibodies were produced by intraperitoneal injection of the Lu 4B5 hybridoma cells into pristine primed Balb/C mice. The mice were sacrificed, the ascites were harvested, and the antibodies were isolated by Protein A chromatography in accordance with the procedure of P. Ey et al., Isolation of pure $IgG_1$, $IgG_{2a}$, and $IgG_{2b}$ immunoglobulins from mouse serum using Protein A Sepharose, 15 Immunochemistry 429 (1978).

EXAMPLE 4

## Partial Purification of the 4B5 Antigen With Immobilized Lu4B5 Antibody

A large bank of bronchial wash specimens collected from patients with lung cancer, and esophageal cancer served as a source of the antigen. The antigen was concentrated by ammonium sulfate precipitation (50-75% salt concentration) from a pool of ten bronchial washes known to contain large amounts of antigen. The precipitated material was reconstituted with phosphate buffered saline (PBS), then dialyzed against PBS with multiple changes of the dialyzate to remove the excess salt. The concentrated antigen was further purified by passage over a 2.5 x 17 centimeter S400 gel filtration column (Pharmacia, Piscataway, New Jersey). 2 milliliter samples were collected from the column and assayed by an ELISA system, utilizing an indirect detection of antigen with goat anti-murine antibody tagged with hoarse-radish peroxidase (Kirkegaard & Perry Labs, Gaithersburg, Maryland). The fractions containing the 4B5 antigen were collected, dialyzed against MilliQ and concentrated by lyophilization. This fraction was then passed over an Affi-gel (Bio-Rad, Richmond, California) immunoaffinity column coupled to the 4B5 antibody. Coupling of the antibody to the solid support was carried out using 0.1M sodium bicarbonate coupling buffer (pH 8.3). This method gave about 75% coupling efficiency. The gel was equilibrated with 100 mM phosphate buffer, pH 6.8 at 4°C. After the partially purified antigen was bound to the column, the bed was washed with 10 volumes of detergent (0.2% Nonidet P-40 in Phosphate buffer) to remove lipids which non-specifically bound to the antigen and column. In addition, the column was washed with 10 volumes of 0.5 M NaCl in Phosphate buffer, to remove contaminants non-specifically bound to the antigen and column by ionic interactions. The column was re-equilibrated with 100 mM phosphate buffer, pH 6.8, and then the antigen was eluted with 1.0 M ammonium hydroxide, 15% ethylene glycol, pH 11.5. Fractions from the affinity column were neutralized with glacial acetic acid. This eluate was dialyzed against MilliQ to remove the elution reagents and lyophilized to concentrate the final product.

EXAMPLE 5

## SDS-PAGE of 4B5 Antigen

Numerous bronchial washes were pooled and partially purified in the manner described in Example 4 above to yield the 4B5 antigen. This purified product was subjected to conventional SDS:Polyacrylamide gel electrophoresis (SDS-PAGE) essentially according to the method of Laemmli (U. Laemmli, 227 Nature 680 (1970)). The sample buffer was run both with and without sulfhydryl reducing agents, and was heated for thirty seconds prior to placement on the gel. The antigen was electrophoresed into a 4% polyacrylamide resolving gel. The antigen was transferred to nitrocellulose (Schleicher & Schuell, Keene, New Hampshire) by electroblotting (100 V, 3 h, 4C) in 25 mM Tris, 192 mM glycine buffer, 20% v/v methanol, pH 8.3. Detection of the protein was accomplished by immunoblotting using the ABC-Alkaline Phosphatase Kit (Vector Labs, Burlingame, California). Briefly, the nitrocellulose is blocked with 3% gelatin in 0.1% (v/v) Tween 20 in Tris-buffered saline (100 mM Tris, .5 M NaCl, pH 7.5). After blocking the nitrocellulose is exposed to Lu 4B5 antibody, followed by biotinylated antibody for thirty minutes. The protein is then exposed to an avidin/alkaline phosphatase complex for thirty minutes. A black reaction product is produced in the final step with incubation of the membrane with the Alkaline Phosphatase substrate (Vector Labs, Burlingame, California). Each immunoblot contained at least one negative control lane, immunoblotted with MPOC/21.

EXAMPLE 6

## Immunohistochemical Analysis of Fresh Frozen Tissue

Surgically resected target tissues were immediately snap frozen in liquid nitrogen and stored until use at -70 degrees Celsius. Frozen tissue was embedded and mounted in OCT compound (Miles Laboratories, Elkhart, IL) prior to sectioning. Frozen sections of 4-6 microns were cut (AO Microtome cryostat, Buffalo, NY) at -16 degrees Celsius and placed on 0.5% gelatin/0.5% potassium dichromate (Fisher Chemical, Fair Lawn, NJ; Sigma Chemical Co., St. Louis, MO, respectively) precoated glass slides (Triangle Biomedical Sciences, Durham, NC). Slides were air-dried overnight and acetone-fixed (Mallinckrodt, Paris, KY) for 10 minutes at room temperature. Slides were washed 3 times in phosphate-buffered saline (PBS) containing 0.01% bovine serum albumin (BSA; type IV, globulin-free, Sigma). Slides were incubated in 10% normal horse serum (J.R. Scientifics, Ocala, FL) diluted in PBS for 15 minutes at room temperature. Slides were drained of excess serum and wiped. Slides were then incubated with monoclonal $IgG_1$ antibody Lu4B5 prepared in accordance with Example 3 above (0.632 mg/ml diluted 1:1000 in PBS/BSA 2%) for 60 minutes at room temperature. To assess nonspecific immunoreactivity, parallel control slides were incubated with purified murine IgG$_1$ (0.359 mg/ml diluted 1:100 in PBS/BSA 2%; Coulter Immunology, Hialeah, FL) for 60 minutes at room temperature. Slides were washed 3 times in PBS/BSA 0.01% to remove unbound antibody. Slides were then incubated with horse anti-mouse biotinylated IgG (1.5 mg/ml diluted 1:100 in PBS/BSA 2%; Vector Laboratories, Burlingame, CA) for 30 minutes at room temperature. Slides were washed 3 times in PBS/BSA 0.01% to remove unbound antibody. Slides were then incubated in Elite solutions A and B (Vectastain; Vector Laboratories) per manufacturers instructions for 30 minutes at room temperature. Slides were washed 3 times in PBS/BSA 0.01%, and subsequently developed using diaminobenzedine solution (100 mg 3',3-diaminobenzedine tetrahydrochloride diluted in 200 ml of Tris-Hydrochloride, pH 7.4; precleared by filtration, and activated immediately prior to use with 2 ml 0.6% hydrogen peroxide; Sigma) for 4 minutes at room temperature. Slides were washed in running tap water for 20 minutes, and counterstained with Gills 3 hematoxylin (Lerner Laboratories, Pittsburgh, PA) for 2 minutes. Slides were washed in running tap water to remove excess counterstain, and dehydrated through successive grades (70%, 95%, 95%, 100%, 100%) of ethanol (Aapar Chemical, Shelbyville, KY). Slides were cleared in 2 changes of xylene (Mallinckrodt), and coverslipped in Permount (Scientific Products, McGaw, IL).

The slides were evaluated independently by two investigators. The overall distribution, intensity, and cellular localization of diaminobenzedine deposition was recorded. The intensity of immunostaining was graded from 0 (no reaction or immunostaining of equal relative intensity to control slides) to ++++ (immunostaining which obscures the nucleus). Positively stained slides were scored as + or greater. Representative slides were photographed by using an Olympus BH-2 light microscope (Olympus Corporation, Lake Success, NY) top-mounted with a Nikon N-2000 SLR camera (Nikon, Garden City, NY).

EXAMPLE 7

## Immunoreactivity of Monoclonal Antibody Lu4B5 in Fresh Frozen Pulmonary Tissue

To assess immunoreactivity of the Lu4B5 antibody against primary lung cancer and adjacent lung tissue, fresh frozen tissue was analyzed by immunohistochemical techniques as outlined in Example 6 above. The data is summarized in Table 1 below.

### TABLE 1

| Tissue/Site | No. Positive Cases/ Total Cases Examined |
|---|---|
| adenocarcinoma/tumor | 1/4 |
| adenocarcinoma/bronchial margin | 1/3 |
| squamous cell carcinoma/tumor | 0/4 |
| squamous cell carcinoma/bronchial margin | 3/4 |
| normal lung/parenchyma | 0/5 |
| normal lung/bronchus . | 0/2 |

Immunohistochemical localization of the Lu4B5 antibody was found in a limited series of frozen tissue to bind focally to normal appearing bronchial mucosa at the surgical bronchial margins from cases of non-small cell lung carcinoma. Cellular localization of diaminobenzedine deposition was evident above the basal nuclei of mucus-secreting goblet cells. Immunoreactivity was also noted in secreted luminal mucin and cilia. Immunoreactivity was further noted in focal areas of peribronchial glands beneath the mucosa. Tumor cells rarely elaborated immunoreactive material. Generally, cellular immunoreactivity was most intense in the peritumor mucosa and peribronchial glandular structures, with an apparent gradient toward lessening immunoreactivity in similar structures distant from the tumor. Normal lung parenchyma (alveolar septae, pneumocytes) or bronchi from cases without lung carcinoma failed to react with the Lu4B5 antibody.

## EXAMPLE 8

### Immunoreactivity of Monoclonal Antibody Lu4B5 in Fresh Frozen Breast Tissue

The immunoreactivity of the Lu4B5 antibody against normal, benign, and malignant breast disease was studied by immunohistochemical techniques as outlined in Example 6 above. The data is summarized in Table 2.

### TABLE 2

| Tissue/Site | No. Positive Cases/ Total Cases Examined |
|---|---|
| normal | 0/2 |
| fibroadenoma | 3/5 |
| carcinoma/tumor cells and/or mucin | 14/41 |
| carcinoma/adjacent ductal cells and/or mucin | 6/6 |
| carcinoma/distant ductal cells and/or mucin | 3/6 |

The Lu4B5 antibody appears to recognize a secreted product of morphologically normal appearing mucosa in the peritumor region of non-small cell lung carcinoma. Furthermore, this product is rarely secreted by the neoplastic cells. Breast tissue was examined for similar immunoreactivity. The data suggests that ductal structures from biopsy-proven normal tissue fail to elaborate immunoreactive material. On the other hand, breast carcinoma cells do elaborate immunoreactive material in approximately 30% of cases examined. Ductal structures in the peritumor region were noted to secrete immunoreactive material in all cases examined; however, the frequency of more distant ductal cell secretion of immunoreactive material was reduced by 50%. Furthermore, in cases of fibroadenoma, elaboration of immunoreactive material was also noted. These data suggest a similar pattern of Lu4B5 reactive material in the breast as had been noted in cases of non-small cell pulmonary carcinoma. It appears that the Lu4B5 antibody reacts with a secreted product in normal breast ducts within the peritumor region, and that this marker had herald premalignant changes in mucosal cells prior to malignant transformation. Lu4B5 immunoreactivity may not be limited solely to the identification of malignancy in pulmonary carcinoma.

## EXAMPLE 9

### Immunohistochemical Analysis of Paraffin-Embedded Tissue

Cassettes containing paraffin-embedded tissue were mounted on a Reichert-Jung Histocut (Heidelberg, West Germany). Tissue sections of 4-6 microns were cut and placed on Histostick (Dakopak, Santa Barbara, CA) precoated glass slides (Triangle Biomedical Sciences) The slides were baked at 56 degrees Celsius for 60 minutes and subsequently cooled to room temperature, followed by dewaxing through 3 changes of xylene (Mallinckrodt). Slides were then cleared through 3 changes of absolute ethanol (Aapar Chemical), and incubated in methanol/hydrogen peroxide solution (3.3 ml 30% hydrogen peroxide in 200 ml methanol; Sigma, Mallinckrodt, respectively) for 30 minutes at room temperature. Slides were gradually equilibrated to deionized water and rinsed for 10 minutes. Slides were then washed 3 times in phosphate-buffered saline (PBS) containing 0.01% bovine serum albumin (BSA; type IV, globulin-free; Sigma). Slides were incubated in 20% normal horse serum (J.R. Scientifics) diluted in PBS for 20 minutes at room temperature. Slides were drained of excess serum and wiped. Slides were then incubated with monoclonal $IgG_1$ antibody Lu4B5 (0.632 mg/ml diluted 1:1000 in PBS/BSA 2%) for 90 minutes at room temperature. To assess nonspecific immunoreactivity, parallel control slides were incubated with purified murine $IgG_1$ (0.359 mg/ml diluted 1:100 in PBS/BSA 2%; Coulter) for 90 minutes at room temperature. Slides were washed 3 times in PBS/BSA 0.01% to remove unbound antibody. Slides were then incubated with horse anti-mouse biotinylated IgG (1.5 mg/ml diluted 1:100 in PBS/BSA 2%; Vector Laboratories) for 45 minutes at room temperature. Slides were washed 3 times in PBS/BSA 0.01% to remove unbound antibody. Slides were then incubated in Elite solutions A and B (Vectastain; Vector Laboratories) per manufacturers instructions for 45 minutes at room temperature. Slides were washed 3 times in PBS/BSA 0.01%, and subsequently developed using diaminobenzedine solution (100 mg 3',3-diaminobenzedine tetrahydrochloride; diluted in 200 ml of Tris-Hydrochloride, pH 7.4; precleared by filtration, and activated immediately prior to use with 2 ml 0.6% hydrogen peroxide; Sigma) for 4 minutes at room temperature. Slides were washed in running tap water for 20 minutes, and counterstained with Gills 3 hematoxylin (Lerner Laboratories) for 2 minutes. Slides were washed in running tap water to remove excess counterstain, and dehydrated through successive grades (70%, 95%, 95%, 100%, 100%) of ethanol (Aapar Chemical). Slides were cleared in 2 changes of xylene (Mallinckrodt), and coverslipped in Permount (Scientific Products).

The slides were evaluated independently by two investigators. The overall distribution, intensity, and cellular localization of diaminobenzedine deposition was recorded. The intensity of immunostaining was graded from 0 (no reaction or immunostaining of equal relative intensity to control slides) to ++++ (immunostaining which obscures the nucleus). Positively stained slides were scored as + or greater. Representative slides were photographed by using an Olympus BH-2 light microscope (Olympus Corporation) top-mounted with a Nikon N-2000 SLR camera (Nikon).

EXAMPLE 10

## Immunohistochemical Localization of Monoclonal Antibody Lu4B5 Immunoreactivity in Paraffin-Embedded Pulmonary Tissue

The immunolocalization of the Lu4B5 reactive moiety was performed by the immunohistochemical techniques described in Example 9 above. Tumor sections were obtained with accompanying surgical bronchial margins in 57 of 60 cases of primary lung cancer. The results are summarized in Table 3.

**TABLE 3**

| Diagnosis/Site | No. Positive Cases/ Total No. Examined |
|---|---|
| non-small cell lung cancer/tumor | 8/60 |
| non-small cell lung cancer/bronchial margin | 41/57 |
| metastatic cancer to lung/tumor | 0/20 |
| metastatic cancer to lung/bronchial margin | 1/20 |
| normal lung/parenchyma | 0/43 |
| normal lung/bronchus | 10/43 |

The Lu4B5 immunoreactive substrate was localized to morphologically normal appearing bronchial mucosa and submucosal glandular structures in the peritumor subsegmental bronchi or bronchioles at a frequency of 72%. Conversely, neoplastic cells rarely elaborate immunoreactive material. Immunostaining was demonstrated within the cytoplasm of goblet cells as well as at the ciliary mucosal layer and intraluminally. In general, immunostaining is diminished in both relative intensity and area at sites more distant from the peritumor region. Conversely, only 17% of bronchial mucosa or submucosal glands demonstrated immunostaining in either metastatic or normal cases. Immunostaining was generally of less overall intensity with less mucosal area stained when compared to the malignant cases. These findings confirm the hypothesis that immunoreactive material is elaborated into tracheobronchial secretions, and should therefore be detectable in either bronchial washings and/or sputum. Furthermore, immunoreactive material should be identified in the majority of patients with pulmonary carcinoma, and in at least some patients at risk to develop pulmonary carcinoma.

EXAMPLE 11

**Immunoreactivity of Monoclonal Antibody Lu4B5 with Malignant and Normal Tissue**

The immunoreactivity of the Lu4B5 antibody against multiple sites of both neoplastic and normal paraffin-embedded tissue (Xenetics, Irvine, CA) was assessed by immunohistochemical techniques as described in Example 9. The data is summarized in Table 4 below.

**TABLE 4**

| Tissue Type | Number Positive/ Number Examined |
|---|---|
| **Malignant** | |
| melanoma | 0/10 |
| lymphoma | 0/8 |
| carcinoid | 0/9 |
| adenocarcinoma | 1/30 |
| sarcoma | 0/10 |
| colon carcinoma | 4/35 |
| undifferentiated carcinoma | 0/8 |
| **Normal** | |
| testis/ovary | 0/9 |
| gastrointestinal | 2/9 |
| spleen | 0/9 |
| pancreas | 1/10 |
| cardiac | 0/9 |
| renal | 0/8 |
| hepatic | 0/10 |
| pulmonary | 0/2 |
| adrenal | 0/10 |
| thyroid | 0/11 |
| colonic adenoma | 0/3 |
| colon | 1/18 |

The Lu4B5 antibody appears to have minimal cross-reactivity with solid organs and tumor tissue. The majority of cross-reactivity appears localized to the gastrointestinal tract, and specifically colonic mucosa. Immunostaining was noted in 4 cases of colon carcinoma, and appeared to be localized to well-differentiated glandular elements of the tumors. Staining was calyceal with reactivity extending to secreted material within the lumen of the glandular structures. Similar staining patterns were noted in the single case of normal colonic mucosa, and in 2 cases of small bowel mucosal staining. Finally, a single normal pancreatic duct was noted to be strongly positive for Lu4B5 reactivity. Although minimal, Lu4B5 cross-reactivity appears limited to mucous-secreting glandular structures within the gastrointestinal tract.

EXAMPLE 12

**Immunodrop Binding Assay**

Bronchial washing specimens were obtained from patients undergoing diagnostic bronchoscopy. Washings were obtained in all cases by instillation of approximately 20 ml of 0.9% sodium chloride. Samples were centrifuged at 2,000 rpm to remove suspended cellular material and debris. The clarified supernatants were frozen and stored at -70 degrees Celsius until use. Following rapid thawing, bronchial washings were vortexed 30 seconds and centrifuged at 10,000 rpm for 60 seconds. An aliquot of supernatant (0.10 ml) was placed onto an uncoated glass slide (Triangle Biomedical Sciences) and air-dried overnight at room temperature, or alternatively, dried at 37 degrees Celsius for 60 minutes. Slides were washed in phosphate-buffered saline (PBS) containing 0.01% bovine serum albumin (BSA; type IV, globulin-free, Sigma). Slides were then preincubated 15 minutes in 5% normal horse serum (Coulter) diluted in PBS/BSA 2%. Slides were then drained of excess serum and wiped. Slides were incubated with monoclonal $IgG_1$ antibody Lu4B5 (0.632 mg/ml diluted 1:500 in PBS/BSA 2%) for 60 minutes at room temperature. To assess nonspecific binding, parallel control slides were incubated with purified murine $IgG_1$ (0.359 mg/ml diluted 1:100 in PBS/BSA 2%; Coulter) for 60 minutes at room temperature. Slides were washed 3 times in PBS/BSA 0.01% to remove unbound antibody. Slides were then incubated in horse anti-mouse biotinylated IgG (1.5 mg/ml diluted 1:100 in PBS/BSA 2%; Vector Laboratories) for 30 minutes at room temperature. Slides were washed 3 times in PBS/BSA 0.01%. Slides were then incubated in Elite solutions A and B (Vectastain; Vector Laboratories) per manufacturer instructions for 30 minutes at room temperature. Slides were washed 3 times in PBS/BSA 0.01%, and developed using diaminobenzidine solution (100 mg 3',3-diaminobenzidine tetrahydrochloride diluted in 200 ml Tris-Hydrochloride, pH 7.4; precleared by filtration, and activated immediately prior to use with 2 ml 0.6% hydrogen peroxide solution; Sigma) for 4 minutes at room temperature. Slides were washed 3 times in PBS/BSA 0.01%, and counterstained in Gills 3 hematoxylin (Lerner Laboratories) for 2 minutes at room temperature. Slides were then washed in running tap water to remove excess counterstain, and dehydrated through successive (75%, 95%, 95%, 100%, 100%) grades of ethanol (Aapar). Slides were cleared in 2 changes of xylene (Mallinckrodt), and coverslipped in Permount (Scientific Products).

Slides were examined for diaminobenzidine deposition within the sample, most notably at the outer edge of the fixed mucinous material. Positively stained slides demonstrated immunostaining of greater intensity than parallel control slides. Representative slides were photographed using an Olympus BH-2 light microscope (Olympus Corporation) with a top-mounted Nikon N-2000 SLR camera (Nikon).

EXAMPLE 13

**Identification of Patients with Pulmonary Carcinoma by the Immunodrop Binding Assay**

It is believed that the most abundant source of Lu4B5 antigenic material is located in the bronchial lumen of lung cancer patients within the peritumor region. This assumption was based on immunolocalization by the Lu4B5 monoclonal antibody in both fresh frozen and paraffin-embedded tissue by immunohistochemistry. A prototypical assay, the immunodrop binding assay, was developed as a rapid screening method for identifying Lu4B5 immunoreactivity from bronchial washing specimens. The method is described in Example 12 above. The data is summarized in Table 5.

**TABLE 5**

| Diagnosis | Number Positive Cases/ Total Cases Examined |
|---|---|
| non-small cell lung carcinoma | 37/57 |
| small cell lung carcinoma | 3/13 |
| esophageal carcinoma | 4/7 |
| metastatic cancer to lung | 3/15 |
| benign lung disease | 8/78 |

Elaboration of Lu4B5 immunoreactive material was identified by the immunodrop binding assay in 65% of bronchial washing specimens from patients with non-small cell lung carcinoma. Conversely, immunoreactive material was identified in only 10% and 20% of bronchial washings from patients with metastatic lung cancer and benign lung disease, respectively. Additionally, 57% of patients with esophageal carcinoma also demonstrated Lu4B5 immunoreactive material. These data suggest that the Lu4B5 monoclonal antibody, as used in the immunodrop binding assay, may specifically identify patients with non-small cell lung carcinoma. This immunospecificity may not be solely limited to pulmonary carcinoma, but may be extended to patients with esophageal carcinoma by the data shown.

EXAMPLE 14

## Quantitation of Tracheobronchial Mucin

This quantitative colorimetric precipitation assay was adapted from Whiteman, P., *Biochem. J.*, 131:351-357, (1973), modified from Hall, R., et al., *Biochem. Soc. Trans.*, 8:72, (1980), and used to estimate quantitatively tracheobronchial mucin content in bronchial washing samples obtained at bronchoscopy. Individual samples were vortexed 30 seconds and centrifuged at 10,000 rpm for 1 minute. Mucin standards were prepared from a 1 mg/ml stock solution of bovine submaxillary mucin (type I; Sigma). Aliquots (50 microliters) of clarified bronchial washing supernatants and known standards were transferred to 1.5 ml microfuge tubes. One ml of Alcian Blue reagent (0.1% w/v Alcian Blue 8GX diluted in 50 mM sodium acetate/50 mM magnesium chloride, pH 5.8; Sigma) was added to each sample. Samples were vortexed and incubated at room temperature for 2-4 hours. The suspended blue precipitate was pelleted at 10,000 rpm for 10 minutes, and supernatant discarded. Pellets were washed 3 times in 50 mM sodium acetate/50 mM magnesium chloride solution. The precipitate was resuspended in 1.0 ml dimethylsulfoxide (DMSO; Fisher). Samples were sonicated with 3 successive 10 second bursts to further disrupt and solubilize the precipitate. Samples were then vortexed vigorously. DMSO blanks were read spectrophotometrically at 620 nm (Gilford Instruments, Model 260, Oberlin, OH). Standard mucin preparations were measured, and standard curves were generated. The mucin content of clinical samples was then determined from the standard curves. A standardized (50 micrograms) amount of total mucin per sample was used in standardized binding assays.

EXAMPLE 15

## Nitrocellulose Filter Immunoassay

Bronchial washing specimens were obtained from patients undergoing diagnostic bronchoscopy. Washings were obtained in all cases by instillation of approximately 20 ml of 0.9% sodium chloride. Samples were centrifuged at 2,000 rpm to remove suspended cellular material and debris. The clarified supernatants were frozen and stored at -70 degrees Celsius until use. Following rapid thawing, bronchial washings were vortexed 30 seconds and centrifuged at 10,000 rpm for 60 seconds. An aliquot of supernatant equal to 50 micrograms of mucin per sample as measured by Alcian Blue colorimetric precipitation in accordance with Example 14 above was loaded per well into parallel 96-well dot blot manifolds (Schleicher and Schuell, Keene, NH) prefitted with BA85 0.45 micron nitrocellulose membranes (Schleicher and Schuell). Spotted samples were air-dried in the manifold by blower. Membranes were preincubated in 100 ml of 0.3% hydrogen peroxide solution (Sigma) for 30 minutes. Membranes were then incubated in TTBS (0.1% Tween 20 in 100 mM Tris-Hydrochloride, 0.5 M NaCl, pH 7.5; Sigma) for 30 minutes at room temperature. Membranes were subsequently incubated with monoclonal $IgG_1$ antibody Lu4B5 (0.632 mg/ml diluted 1:500 in TTBS) for 30 minutes at room temperature. To assess nonspecific binding, parallel control slides were incubated with purified murine $IgG_1$ (0.359 mg/ml diluted 1:100 in TTBS; Coulter) for 30 minutes at room temperature. Membranes were washed 4 times in TTBS to remove unbound antibody. Membranes were then incubated with horse anti-mouse biotinylated IgG (1.5 mg/ml diluted 1:100 in TTBS; . Coulter) for 30 minutes at room temperature. Membranes were washed 4 times in TTBS, and then incubated in Elite solutions A and B (Vectastain; Vector Laboratories) diluted in 5 ml TTBS for 30 minutes at room temperature. Membranes were washed 4 times in TTBS, then subsequently developed using diaminobenzidine solution (100 mg 3',3-diaminobenzidine tetrahydrochloride diluted in 200 ml Tris-Hydrochloride, pH 7.4; precleared by filtration, and activated immediately prior to use with 2 ml 0.6% hydrogen peroxide solution; Sigma) for 2-4 minutes at room temperature. Membranes were washed 4 times in TTBS and air-dried.

Immunoreactivity was assessed by diaminobenzidine deposition on the nitrocellulose membranes. Positive immunostaining was scored from 0 (no immunostaining or staining equal to control immunoreactivity) to ++++ (heavy immunostaining).

EXAMPLE 16

### Identification of Patients with Pulmonary Carcimona by the Standardized Nitrocellulose Immunoassay using the Lu4B5 Monoclonal Antibody

Whereas the immunodrop binding assay utilized nonstandardized mucin samples for immunoreactivity, the nitrocellulose filter immunoassay was devised to use equivalent amounts of total mucin per sample tested. The protocols for the measurement of total mucin and the standardized nitrocellulose filter immunoassay are given in Examples 14 and 15 above. The data are summarized in Table 6.

**TABLE 6**

| Diagnosis | Number Positive Cases/ Number Cases Examined |
|---|---|
| non-small cell lung carcinoma | 30/57 |
| small cell lung carcinoma | 6/13 |
| esophageal carcinoma | 4/7 |
| metastatic cancer to lung | 3/15 |
| benign lung disease | 12/78 |

The Lu4B5 immunoreactive material was identified in 53% of bronchial washings obtained from patients with non-small cell lung cancer. Additionally, Lu4B5 immunoreactive material was identified in 46% of washings from patients with small cell lung carcinoma. The frequency of detection of Lu4B5 immunoreactive material in cases of esophageal carcinoma and metastatic cancer to the lung remained at 57% and 20%, respectively. Immunospecificity was reduced by 5% in this immunoassay in those cases of benign lung disease. This immunoassay demonstrates slightly diminished sensitivity and specificity for the identification of patients with primary pulmonary carcinoma; however, it serves as foundation for a quantitative standardized nitrocellulose-based radioimmunoassay.

EXAMPLE 17

### Nitrocellulose-Based Radioimmunoassay

Bronchial washing specimens were obtained from patients undergoing diagnostic bronchoscopy. Washings were obtained in all cases by instillation of approximately 20 ml of 0.9% sodium chloride. Samples were centrifuged at 2,000 rpm to remove suspended cellular material and debris. The clarified supernatants were frozen and stored at -70 degrees Celsius until use. Following rapid thawing, bronchial washings were vortexed 30 seconds and centrifuged at 10,000 rpm for 60 seconds. An aliquot of supernatant equal to 50 micrograms of mucin per sample as measured by Alcian Blue colorimetric precipitation was loaded per well into parallel 96-well dot blot manifolds (Schleicher and Schuell) prefitted with BA85 0.45 micron nitrocellulose membranes (Schleicher and Schuell). Spotted samples were air-dried in the manifold by blower. Membranes were preincubated in Blotto solution (2% non-fat dry milk, 1% Triton X-100, 50 mM Tris-Hydrochloride, pH 7.5, 10 mM ethylenediamine tetraacetic acid; Sigma) containing 0.1% bovine serum albumin (BSA; type IV, globulin-free; Sigma) for 60 minutes at room temperature under continuous gentle agitation. Membranes were washed 4 times in Blotto solution, then subsequently incubated with monoclonal $IgG_1$ antibody Lu4B5 (0.632 mg/ml diluted 1:500 in Blotto solution) for 30 minutes at room temperature. To assess nonspecific binding, parallel control

13

slides were incubated with purified murine $IgG_1$ (1.0 mg/ml diluted 1:1400 in Blotto solution; Coulter) for 30 minutes at room temperature. Membranes were washed 4 times in Blotto solution to remove unbound antibody. Membranes were then incubated with $10^7$ cpm/membrane of sheep anti-mouse [$^{125}$I]-labeled F(ab')$_2$ Ig fragment (149 uCi/ml: New England Nuclear, Wilmington, DE) for 60 minutes at room temperature. Membranes were washed 4 times in Blotto solution and subsequently air-dried. Individual circles were cut from the membranes and binding quantitated by gamma counting (Packard Instruments, Sterling, VA).

Specific binding was determined by subtracting the nonspecific binding (as represented by cpm bound by murine IgG) from the total binding (as represented by cpm bound by monoclonal antibody Lu4B5).

EXAMPLE 18

### Identification of Patients with Primary Pulmonary Cancer by the Standardized Nitrocellulose Radioimmunoassay using the Lu4B5 Monoclonal Antibody

Quantitative specific binding of Lu4B5 immunoreactive material in bronchial washing specimens was assessed using the standardized nitrocellulose radioimmunoassay configuration as described in Example 17 above. The data is summarized in Tables 7 and 8 below.

## TABLE 7

| Diagnosis | Number Positive Cases/ Total Cases Examined |
|---|---|
| non-small cell lung carcinoma | 26/43 |
| small cell lung carcinoma | 6/9 |
| esophageal carcinoma | 2/4 |
| metastatic cancer to lung | 1/12 |
| benign lung disease | 15/72 |

**TABLE 8**

| Variable | Non-Small Cell | Small Cell | Diagnosis | | |
|---|---|---|---|---|---|
| | | | Esopha-geal | Meta-static | Benign Disease |
| N | 43 | 9 | 4 | 12 | 72 |
| age (median, years) | 64.0 | 61.0 | 66.5 | 56.5 | 56.0 |
| smoking (median, pack-yrs) | 45.0 | 65.0 | 60.0 | 0.0 | 20.0 |
| mucin (median, mg/ml) | 0.35 | 0.36 | 1.37 | 0.24 | 0.38 |
| RIA (median, cpm) | 686 | 651 | 700 | 220 | 217 |

The Lu4B5 immunoreactive moiety was identified in 61% of bronchial washings from patients with non-small carcinoma of the lung using the standardized nitrocellulose radioimmunoassay. Further, 67% of patients with small cell carcinoma elaborated Lu4B5 immunoreactive material. Conversely, 8% and 21% of patients with either metastatic lung cancer or benign pulmonary disease, respectively, secreted immunoreactive material in bronchial washing specimens. Of these "false-positive" patients, 10 of 15 had significant smoking histories, and an additional patient had a significant occupational exposure to a known carcinogen, both situations known to involve an increased risk in the development of lung cancer. These data suggest that the Lu4B5 immunoreactive substance is elaborated and detectable primarily in the bronchial secretions of patients with primary lung carcinoma, and additionally, in the secretions of patients with esophageal carcinoma. The standardized nitrocellulose radioimmunoassay offers a sensitive and relatively specific method of identifying the Lu4B5 immunoreactive moiety in bronchial washings.

EXAMPLE 19

### Double-Determinant Nitrocellulose-Based Radioimmunoassay

Polystyrene flat bottom wells (Immulon 2 Removawell; Dynatech Laboratories; Chantilly, VA) were precoated with 1.0 microgram of either monoclonal $IgG_1$ antibody Lu4B5 (55.4 microliters of 0.632 mg/ml stock diluted in 7 ml of 50 mM Tris-Hydrochloride, pH 7.5 containing 0.1% type IV, globulin-free bovine serum albumin; BSA; Sigma) or purified murine $IgG_1$ (35.0 microliters of 1.0 mg/ml stock diluted in 7 ml of 50 mM Tris-Hydrochloride, pH 7.5/BSA 0.1%) overnight at 4 degrees Celsius. Wells were washed twice with deionized water, and overcoated with 0.40 ml of 1% BSA diluted in 50 mM Tris-Hydrochloride, pH 7.5, for 60 minutes at room temperature. Wells were then washed 5 times with deionized water, loaded with 50 micrograms of mucin per well, and incubated 2 hours at room temperature, or alternatively, at 4 degrees overnight. Wells were again washed 5 times with deionized water to remove unbound material. Radiolabeled [$^{125}$I]-Lu4B5 (0.20 ml of a 1:200 dilution of 190 uCi/ml stock solution) was added to each well and incubated for 2 hours at room temperature. Wells were washed 5 times with deionized water to remove unbound radiolabeled antibody. Individual wells were then separated and binding quantitated by gamma counting (Packard Instruments).

Specific binding was determined by subtracting the nonspecific binding (as represented by cpm bound by murine IgG) from the total binding (as represented by cpm bound by monoclonal antibody Lu4B5).

EXAMPLE 20

## Immunoreactivity of Monoclonal Antibody Lu4B5 in a Double-Determinant Radioimmunoassay

The ability of monoclonal antibody Lu4B5 to maintain sensitive and specific immunoreactivity was assessed using the nitrocellulose-based double-determinant "sandwich" radioimmunoassay described in Example 19. Specific binding was compared to immunoreactivity as assessed previously by nitrocellulose-based immunoassays. Additionally, antigen titration and prolonged incubation were both assessed. The results are summarized in Table 9 below.

### TABLE 9

| Sample | Nitrocellulose Immunoassay | Nitrocellulose RIA (cpm) | Double-Determinant |
|---|---|---|---|
| NSCLC-1 | +++ | 1555 | 4584 |
| NSCLC-2 | +++ | 4380 | 2441 |
| NSCLC-3 | ++ | 733 | 1938 |
| NSCLC-4 | ++ | 3867 | 2056 |
| NSCLC-5 | + | 123 | 2334 |
| NSCLC-6 | + | ND | 1577 |
| SCLC-1 | +++ | 3534 | 2394 |
| SCLC-1 (16 hr) | ND | ND | 789 |
| SCLC-1 (1:2) | ND | ND | 1326 |
| SCLC-1 (1:4) | ND | ND | 976 |
| SCLC-1 (1:8) | ND | ND | 595 |
| SCLC-1 (1:16) | ND | ND | 347 |
| SCLC-1 (1:32) | ND | ND | -126 |
| SCLC-1 (1:64) | ND | ND | -120 |
| SCLC-1 (1:128) | ND | ND | 32 |
| ESOPH-1 | +++ | 1652 | 3573 |
| BENIGN-1 | 0 | 59 | -123 |
| BENIGN-2 | 0 | 300 | -257 |
| BENIGN-3 | 0 | 303 | 295 |
| BENIGN-4 | 0 | 87 | 127 |
| BENIGN-5 | 0 | 130 | -57 |
| BENIGN-6 | +++ | 2858 | 2931 |
| BENIGN-7 | +++ | 2116 | 2156 |
| BENIGN-7 (16 hr) | ND | ND | 4293 |
| BUFFER-1 | 0 | 201 | -152 |
| BUFFER-1 (16 hr) | ND | ND | 69 |
| BUFFER-2 | 0 | 184 | -462 |
| BUFFER-2 (16 hr) | ND | ND | -36 |

The monoclonal antibody Lu4B5 reacted specifically with the bronchial washings from 6 cases of non-small cell lung carcinoma (NSCLC), 1 case of small cell lung carcinoma (SCLC), and 1 case of esophageal carcinoma (ESOPH) in a double-determinant "sandwich" immunoassay format. Furthermore, in 5 cases of benign pulmonary disease, minimal specific binding was quantitated. In 2 known false-positive reacting benign cases, the double-determinant assay continued significant immunoreactivity. It appears, therefore, that the antigen recognized by antibody Lu4B5 is multiply expressed, or that multiple cross-reactive epitopes exist per antigenic substrate which allow binding in a "sandwich" format. Furthermore, there appears to be no loss of reactivity when compared to prior indirect, non-sandwich immunoassays, and this immunoreactivity correlates favorably with these prior assays. Prolonged incubation does not appear to enhance immunoreactivity. Finally, immunoreactivity is reduced in a linear manner with serial dilutions of the antigenic material (bronchial washing).

The foregoing examples are illustrative of the present invention, and are not to be construed as limiting thereof. The invention is defined by the following claims, with equivalents of the claims to be included therein.

## Claims

1. A monoclonal antibody selected from the group consisting of monoclonal antibody Lu4B5, monoclonal antibodies which bind to the antigen bound by monoclonal antibody Lu4B5, and fragments thereof which bind to the antigen bound by monoclonal antibody Lu4B5.

2. A monoclonal antibody according to claim 1 selected from the group consisting of monoclonal antibody Lu4B5, monoclonal antibodies which bind to the epitope bound by monoclonal antibody Lu4B5, and fragments thereof which bind to the epitope bound by monoclonal antibody Lu4B5.

3. A monoclonal antibody according to claim 1, wherein said fragments thereof are selected from the group consisting of Fab fragments, $F(ab')_2$ fragments, and Fv fragments.

4. A monoclonal antibody according to claim 1 conjugated to a label capable of producing a detectable signal.

5. A monoclonal antibody according to claim 3 wherein said label is selected from the group consisting of free radicals, fluorescent dyes, enzymes, radioisotopes, bacteriophages, and coenzymes.

6. A cell line capable of producing a monoclonal antibody according to claim 1.

7. A cell line according to claim 6, which cell line is selected from the group consisting of a hybridoma cell line and an *Escherichia coli* cell line.

8. A method for detecting the presence of cancer, comprising contacting a sample from a subject with a monoclonal antibody according to claim 1 under conditions permitting said monoclonal antibody to form a reaction product, and detecting the presence or absence of said reaction product.

9. A method according to claim 8, wherein said sample is a mucus secretion.

10. A method according to claim 8, wherein said sample is selected from the group consisting of a respiratory tract mucus secretion and an aero-digestive tract mucous secretion.

11. A method of making antibodies capable of detecting cancer or a risk of cancer in a subject, comprising:
    collecting mucus secretions from a cancerous tissue in a subject; then
    providing a non-cellular phase produced from the collected mucus secretions; then
    generating a plurality of antibodies against antigens contained in the non-cellular phase; and then
    selecting antibodies from said plurality of antibodies, which selected antibodies are capable of selectively detecting cancer or a risk of cancer in a subject.

12. A method according to claim 11, wherein said step of generating a plurality of antibodies comprises the steps of:
    administering the non-cellular phase to an animal recipient;
    collecting spleen cells from the animal recipient;
    fusing the collected spleen cells with immortal cells to produce hybridoma cells; and then
    selecting a hybridoma cell which produces an antibody which is capable of selectively detecting cancer or a risk of cancer in a subject.

13. A method according to claim 11, wherein the cancerous tissue is epithelial tissue.

14. A method according to claim 11, wherein the mucus secretions are selected from the group consisting of respiratory tract secretions and aerodigestive tract secretions.

**15.** A method according to claim 11, wherein the non-cellular phase is mucin-containing.

**16.** A test kit comprising: (a) an antibody according to claim 1, and (b) a conjugate of a specific binding partner for the antibody of claim 1 and a label capable of producing a detectable signal.

EP 0 454 338 A1

**European Patent Office**

# EUROPEAN SEARCH REPORT

EP 91 30 3348

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,P | WO-A-9 005 178 (NORTHWESTERN UNIVERSITY) * Page 5, line 20 – page 8, line 6 * --- | 11 | C 12 P 21/08 C 12 N 5/12 G 01 N 33/577 |
| Y,D | EP-A-0 212 403 (SLOAN KETTERING INSTITUTE FOR CANCER RESEARCH) * Column 7, line 15 – column 15, line 48 * ---- | 8-10 | |
| Y,D | WO-A-8 805 054 (IMPERIAL CANCER RESEARCH TECHNOLOGY LTD) * Page 4, line 1 – page 14, line 14 * --- | 1-16 | |
| Y,D | WO-A-8 603 838 (ONCOGEN) * Page 5, line 1 – page 18, line 12 * ----- | 1-16 | |

| TECHNICAL FIELDS SEARCHED (Int. Cl.5) |
|---|
| C 07 K C 12 P |

The present search report has been drawn up for all claims

| Place of search | Date of completion of the search | Examiner |
|---|---|---|
| THE HAGUE | 24-07-1991 | REMPP G.L.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

19